# EUROPÄISCHE PATENTSCHRIFT

(11) **EP 3 525 734 B1**
(45) Veröffentlichungstag und Bekanntmachung des Hinweises auf die Patenterteilung: **02.07.2025**
(21) Anmeldenummer: 17791277.1
(22) Anmeldetag: 17.10.2017
(51) Int. Cl.: A61F 7/00, A61F 7/02

(54) **VORRICHTUNG ZUR KÜHLUNG EINER KÖRPERREGION**
DEVICE FOR COOLING A BODY REGION
DISPOSITIF POUR REFROIDIR UNE RÉGION DU CORPS

(30) Priorität: 17.10.2016 AT 509392016
(43) Veröffentlichungstag der Anmeldung: 21.08.2019
(73) Patentinhaber: AUROX GmbH, 8010 Graz (AT)
(72) Erfinder: Schöggler, Christoph, 8010 Graz (AT)
(74) Vertreter: Isarpatent
(86) Internationale Anmeldenummer: PCT/AT2017/060265
(87) Internationale Veröffentlichungsnummer: WO 2018/071933

(56) Entgegenhaltungen:
- EP-A2- 0 050 473
- WO-A1-2014/059454
- WO-A1-2015/106180
- WO-A1-2015/145471
- WO-A1-2016/160691
- US-A1- 2008 300 529
- US-A1- 2013 116 759
- US-A1- 2016 178 251

## Beschreibung

Die Erfindung betrifft eine Vorrichtung zur Kühlung und/oder Erwärmung einer Körperregion, bevorzugt eines Kopfbereiches, umfassend zumindest ein wärmeleitfähiges Hautkontaktmaterial und zumindest ein Peltierelement, wobei das Peltierelement mit dem Hautkontaktmaterial verbunden ist. Die Erfindung ist durch den beigefügten Anspruch 1 definiert. Ausführungsbeispiele sind in den abhängigen Ansprüchen offenbart.

Jährlich werden viele Tausende Schönheitsoperationen durchgeführt, beispielsweise an der Nase. Nach einer Nasenoperationen wird zur postoperativen Versorgung meist ein 3 mm bis 5 mm dünner Gips über die Nase gelegt. Von Ärzten wird neben einer medikamentösen Therapie auch eine Kühlung sowie Trockenlegung der Nase empfohlen, um eine rasche Schmerzlinderung sowie eine baldige Wund- und Narbenheilung zu erreichen. Zur Kühlung und Linderung von Schwellungen wird häufig ein Eisbeutel verwendet.

Nachteilig bei einer Verwendung eines Eisbeutels ist, dass der Eisbeutel einen Gips oder Verband durchnässt, weshalb dieser öfters getauscht werden muss. Darüber hinaus ist eine Kühltemperatur mit einem Eisbeutel nicht regelbar und zudem meist zu niedrig. Es hat sich herausgestellt, dass für eine Kühlung einer geschwollenen Körperregion wie einer Nase zur Unterstützung der Abheilung eine bestimmte Temperatur nicht unterschritten werden darf. Zu starke, länger andauernde Temperatursenkungen, welche bei einer Verwendung von einem Eisbeutel auftreten, sind nicht erwünscht, da ein Patient negative Auswirkungen wie Schmerzen, Taubheitsgefühle und andere Gefühlstörungen erfahren kann.

Hier setzt die Erfindung an. Aufgabe der Erfindung ist es, eine Vorrichtung der eingangs genannten Art anzugeben, mit welcher eine Körperregion mit geringem Aufwand individuell und effizient kühlbar ist.

Die Aufgabe wird erfindungsgemäß dadurch gelöst, dass bei einer Vorrichtung der eingangs genannten Art ein wärmeleitfähiges Verbindungselement, insbesondere eine Wärmeleitfolie, und ein Wärmeabführelement vorgesehen sind, wobei über das Verbindungselement über eine Außenseite des Peltierelementes abgegebene Wärme auf das Wärmeabführelement verteilbar ist.

Ein mit der Erfindung erzielter Vorteil ist insbesondere darin zu sehen, dass Wärme an einer Außenseite des Peltierelementes mittelbar oder unmittelbar, vorzugsweise durch direkten Kontakt, auf das Verbindungselement übertragbar und über dieses in weiterer Folge auf das Wärmeabführelement verteilbar bzw. übertragbar und anschließend über das Wärmeabführelement nach außen abführbar ist. Alternativ dazu kann das Hautkontaktmaterial mittels Peltierelement erwärmt werden. Somit kann eine bestimmte Körperregion, welche in Kontakt mit dem Hautkontaktmaterial steht, erwärmt werden. Dadurch sind auch unterschiedliche Temperaturverläufe an jedem einzelnen Hautkontaktmaterial ermöglicht. Hierfür kann das Hautkontaktmaterial beispielsweise als Hautkontaktplättchen ausgebildet sein. Eine erfindungsgemäße Vorrichtung kann beispielsweise ein oder mehrere solcher Hautkontaktplättchen umfassen.

Das wärmeleitfähige Verbindungselement ist bevorzugt flexibel sowie aus einem Heatspreadermaterial ausgebildet, beispielsweise aus Grafit oder Kupfer. Das Verbindungselement kann beispielsweise als Wärmeleitfolie wie als Grafitfolie oder Kupferblech ausgebildet. Zumeist ist das Verbindungselement jedoch als flexibles Wärmeleitpad ausgebildet. Eine als Grafitfolie ausgebildete Wärmeleitfolie ist biegsam bzw. flexibel und hauchdünn ausgebildet. Das wärmeleitfähige Verbindungselement eignet sich zum Abführen von Wärme an einer Außenseite des Peltierelementes, wobei dieses zwischen dem zumindest einen Peltierelement und dem z. B. als Aluminiumblech ausgebildeten Wärmeabführelement angeordnet ist. Durch eine leitfähige Wärmeleitfolie bzw. ein Wärmeleitpad oder eine Wärmeleitpaste ist Wärme großflächig weiterverteilbar, insbesondere auf das zur Abführung der Wärme vorgesehenes Wärmeabführelement. Besonders bevorzugt ist das Verbindungselement unmittelbar mit einer Außenseite einer Leiterplatte verbunden. Das wärmeleitfähige Verbindungselement ist insbesondere flächig ausgebildet, sodass dieses einen großen Teil einer Fläche der Vorrichtung umfasst. Eine Querschnittsfläche der Wärmeleitfolie ist zumindest gleich groß wie, vorzugsweise allerdings um ein Vielfaches größer als eine Querschnittsfläche des zumindest einen Peltierelementes. Es kann vorgesehen sein, dass die flächige sowie dünne Wärmeleitfolie von einem ersten Peltierelement bis zu einem nächsten bzw. zweiten reicht. Alternativ kann das wärmeleitfähige Verbindungselement auch als wärmeleitfähige Paste bzw. Wärmeleitpaste ausgebildet sein, welche das Peltierelement mit dem Wärmeabführelement verbindet. Eine Querschnittsfläche einer solchen Paste entspricht dabei im Wesentlichen der Fläche des Peltierelementes. Sind mehrere Peltierelemente vorgesehen, ist zwischen jedem Peltierelement und dem Wärmeabführelement eine wärmeleitfähige Paste vorgesehen. Das Wärmeabführelement ist bevorzugt flächig ausgebildet und erstreckt sich insbesondere über einen Großteil der Vorrichtung.

Erfindungsgemäß ist das Wärmeabführelement biegbar ausgebildet. Hierfür kann beispielsweise vorgesehen sein, dass das Wärmeabführelement aus einem Aluminiumblech ausgebildet ist. Gegebenenfalls können auch zwei oder mehr Wärmeabführelemente vorgesehen sein. Um das Wärmeabführelement flexibel auszubilden, kann vorgesehen sein, dass dieses aus einem flexiblen Material ausgebildet ist. Alternativ dazu kann das Wärmeabführelement mehrteilig ausgebildet sein, wobei die Teile biegsam verbunden sind.

Mit dem Peltierelement ist es möglich, das wärmeleitfähige Hautkontaktmaterial auf eine bestimmte Temperatur zu bringen und damit eine ideale Abheilung einer geschwollenen Körperregion sicherzustellen. Beispielsweise ist eine operierte Nase individuell angepasst kühlbar, insbesondere am Nasenbein, Seitenknorpel, Flügelknorpel sowie Jochbeinareal und an den Wangenknochen. Das Hautkontaktmaterial ist insbesondere aus einem Metall gebildet, bevorzugt aus Aluminium, Kupfer, Titan oder einer entsprechenden Legierung mit einem solchen Metall. Besonders bevorzugt ist das Hautkontaktmaterial aus einer biegsamen metallischen Folie gebildet, beispielsweise einer versilberten Kupferfolie oder einem eloxierten oder silberbeschichteten Aluminiumplättchen. Das Hautkontaktelement kann auch aus einem in einem ersten Schritt versilberten und in einem zweiten Schritt rhodinierten Kupferplättchen oder einem vergoldeten Plättchen gebildet sein. Das Hautkontaktmaterial kann auch aus Palladium oder einem anderen Metall gebildet sein. Alternativ kann es auch günstig sein, wenn das Hautkontaktelement aus mehreren kleinen starren Blechelementen gebildet ist. Dadurch ist das Hautkontaktmaterial einer Form der zu kühlenden Körperregion anpassbar, wodurch die Körperregion in weiterer Folge gleichmäßig kühlbar ist.

Der zu kühlenden Körperregion ist über das Peltierelement Wärme entziehbar. Sollen mehrere Körperregionen wie beispielsweise beide Jochbeinareale gekühlt werden, sind für jede zu kühlende Körperregion ein Hautkontaktmaterial und ein Peltierelement vorgesehen. Sind mehrere Peltierelemente vorgesehen, können diese unabhängig voneinander ansteuerbar sein, wobei die Peltierelemente voneinander abhängig oder unabhängig ausgebildet sind.

Die tragbare Vorrichtung umfasst vorteilhaft einen thermisch konduktiven Schichtaufbau, mit welchem Wärme nach außen abgebbar ist, wobei der Schichtaufbau zumindest teilweise flexibel ausgebildet ist. Dadurch ist die tragbar ausgebildete Vorrichtung flexibel an eine zu kühlende Körperregion anpassbar und Wärme über den Schichtaufbau nach außen abführbar.

Die Vorrichtung ist frei von flüssigem Wärmetransportmittel ausgebildet.

Dadurch wird eine Nässung des zu kühlenden Bereichs und beispielsweise eines Gipses oder anderen Verbandmaterials sowie der Haut und des Haares eines Patienten vermieden. Darüber hinaus ist es günstig, wenn die Vorrichtung frei von Strömungsmaschinen wie Lüfter, Ventilatoren und dergleichen ist.

Es ist von Vorteil, wenn eine Leiterplatte vorgesehen ist. Die Leiterplatte ist insbesondere zumindest teilweise flexibel ausgebildet. Alternativ dazu umfasst diese beispielsweise zwei, drei, vier oder mehr starre Teile, welche mit flexiblen Kabeln oder dergleichen miteinander verbunden sind. Diese umfasst weiter vorteilhaft mehrere elektronische Bauteile und erstreckt sich insbesondere über eine gesamte Fläche der Vorrichtung, wohingegen das Peltierelement kleiner ausgebildet ist. Dadurch ist abzuführende Wärme großflächig verteilbar und damit eine effiziente Kühlung rasch durchführbar. Es ist kein flüssiges Wärmetransportmittel sowie kein Lüfter und/oder Ventilator vorgesehen oder notwendig. Die Vorrichtung ist zur Kühlung einer Körperregion, beispielsweise eines Kopfbereiches oder Nasenbereiches, insbesondere nach einer Operation, ausgebildet. Es ist insbesondere vorteilhaft, wenn die Leiterplatte mit einem oder mehreren thermischen Vias ausgebildet ist, um eine Wärmeableitung durch ein, insbesondere wärmeisolierendes, Leiterplattenmaterial wie beispielsweise FR4 zu ermöglichen. An der Leiterplatte kann überdies eine Abdeckung aus einem wärmeleitfähigen Material, beispielsweise ein dünnes Aluminiumblech, insbesondere mit einer Dicke von etwa 0,2 mm, angeordnet sein, welches Wärme aus den Vias an eine Umgebungsluft ableitet. Des Weiteren kann mit dieser Abdeckung eine glatte, ungefährliche und insbesondere hautverträgliche Oberfläche bereitgestellt sein.

Die Leiterplatte erstreckt sich dabei zweckmäßigerweise von einem Peltierelement bis zum nächsten. Die Leiterplatte kann jedoch auch durch Drahtverbindungen unterbrochen bzw. zwei Teile der Leiterplatte mit einer Drahtverbindung verbunden sein. Des Weiteren kann es vorgesehen sein, dass die Leiterplatte über eine warme Seite des Peltierelementes durchgehend angeordnet ist. Durch die zumindest teilweise flexible Ausbildung derselben ist die Leiterplatte an eine Form der zu kühlenden Körperregion anpassbar, sodass die zu kühlende Körperregion individuell sowie effizient kühlbar ist. Das zumindest eine Peltierelement ist über die Leiterplatte ansteuerbar und regelbar, was eine individuelle Kühlung ermöglicht. Es kann weiter ein flexibles Isoliermaterial vorgesehen sein, welches sich im Wesentlichen über eine gesamte Fläche der Vorrichtung erstreckt und in welchem das Peltierelement eingebettet ist. Das Isoliermaterial ist dazu ausgebildet, das Peltierelement und andere Elemente der Vorrichtung vor Beschädigung und Abnutzung zu schützen. Vorteilhaft ist die Leiterplatte außenseitig am Isoliermaterial angeordnet. Das Hautkontaktmaterial weist bevorzugt eine größere Fläche auf als das Peltierelement und erstreckt sich über eine Unterseite des Isoliermaterials, um in weiterer Folge eine Körperregion zu kühlen. Das Isoliermaterial kann beispielsweise aus Latex, Silicon oder einem anderen weichen Kunststoff oder einer Kombination aus verschiedenen Kunststoffen gebildet sein. Alternativ dazu kann das Isoliermaterial auch aus einem starren Kunststoff gebildet sein oder einen solchen umfassen. Insbesondere ist das Isoliermaterial aus einem thermoplastischen Elastomer wie einem thermoplastischen Polyurethan gebildet. Zur verbesserten Wärmeisolation kann zusätzlich eine Kunststofffolie eingelegt werden, um eine Wärmerückkopplung zu vermeiden. Weiter ist das Hautkontaktmaterial stets so an der Vorrichtung angeordnet, dass dieses mit der zu kühlenden Körperregion unmittelbar in Kontakt ist. Da Temperaturverläufe mit dem Peltierelement exakt steuerbar sind, ist die Vorrichtung zusätzlich für eine leichte Erwärmung verwendbar, beispielsweise bei einer Warm-kalt-Therapie. Die Vorrichtung ist insbesondere so ausgebildet, dass Kühlverläufe möglich sind. Hierfür sind alle Kühlflächen frei regelbar und steuerbar, sodass eine beliebige Kühlkurve an einer gewünschten Stelle angewendet werden kann.

Es ist weiter vorteilhaft, wenn zumindest ein Kühlkörper vorgesehen ist, um die von der Haut abgeführte Wärme durch Wärmeleitung bzw. freie Konvektion an die Luft abzugeben. Der Kühlkörper kann starr bzw. steif oder leicht flexibel ausgebildet sein. Dieser ist mit Vorteil aus einem Metall, insbesondere aus einem Aluminiumblech oder Kupferblech gebildet, welches eine gute Wärmeleitfähigkeit aufweist und nicht vollständig starr ausgebildet ist.

Zweckmäßig ist es, wenn zum Betrieb des Peltierelementes eine Energiequelle vorgesehen ist, wobei die Energiequelle insbesondere über Kabel mit dem Peltierelement verbunden ist. Alternativ oder zusätzlich kann die Energiequelle auch über die Leiterplatte mit dem Peltierelement verbunden sein. Die Energiequelle kann als Akkumulator ausgebildet und über Kabel mittelbar mit dem Peltierelement zur Stromversorgung desselben verbunden sein. Insbesondere ist eine externe Energiequelle vorgesehen. Es kann jedoch auch vorgesehen sein, dass eine Energiequelle wie eine oder mehrere Batterien oder Akkumulatoren in der Vorrichtung eingebettet ist. Kabel des Peltierelementes sind bevorzugt nahe an diesem abgezwickt und elektrisch mit der Leiterplatte verbunden. Es kann insbesondere vorgesehen sein, wenn die Energiequelle in einem Kunststoffgehäuse bzw. Akkumulatorgehäuse, beispielsweise aus einem, bevorzugt hautverträglichen, thermoplastischen Elastomer, eingefasst ist.

Günstig ist es weiter, wenn eine Steuereinheit vorgesehen ist. Die Steuereinheit ist mit dem Peltierelement sowie der Energiequelle verbunden, damit das Peltierelement steuerbar und regelbar ist. Alternativ oder zusätzlich zur Steuereinheit kann eine Regeleinheit vorgesehen sein. Die Steuereinheit und/oder Regeleinheit kann grundsätzlich an einer beliebigen Position angeordnet sein, zweckmäßig ist es jedoch, wenn diese zwischen der Energiequelle und der Vorrichtung angeordnet ist. Die Steuereinheit überwacht und regelt Temperaturen, mit welchen eine Körperregion gekühlt werden soll. Dadurch sind Kühlabläufe beliebig gestaltbar, beispielsweise ist es möglich, eine Intervallkühlung oder eine Kühlung bei gleichbleibender Temperatur einzustellen. Darüber hinaus erlaubt die Steuereinheit eine Vorgabe bestimmter Maximalwerte und/oder Minimalwerte für die Temperatur, sodass ein gewünschter Temperaturbereich für eine individuelle Behandlung einstellbar ist. Es ist somit beispielsweise ein unerwünschtes Unterschreiten einer Minimaltemperatur vermeidbar. Weiter kann ein Patient eine beliebige Behandlungstemperatur individuell auf sein Befinden abstimmen. Temperaturwerte und/oder Temperaturverläufe sind besonders bevorzugt variabel programmierbar bzw. einstellbar. Des Weiteren sind externe Signale vom Körper, Umwelt und/oder Umgebung in einen Programmverlauf miteinbeziehbar. Die Vorrichtung bzw. Steuereinheit kann auch lernfähig ausgebildet sein. Dadurch sind Körperfunktionen wie z. B. ein Sauerstoffgehalt im Blut und/oder ein Hautwiderstand sowie externe Signale wie eine Lichtintensität, eine Lautstärke und/oder Vibrationen überwachbar. Ferner kann so auch ein Einfluss des Wetters überwacht werden. Aufgrund von Temperatur- und/oder Luftdruckänderungen variiert eine Wahrscheinlichkeit für beispielsweise Kopfschmerzen oder allgemein körperliches Wohlbefinden. Eine Ansteuerung der Steuereinheit zur Regelung von speziellen Kühlverläufen erfolgt zweckmäßigerweise über ein Mobiltelefon oder dergleichen, welches z. B. über Bluetooth Low Energy mit der Steuereinheit verbunden ist. Die Steuereinheit und/oder Regeleinheit umfasst zweckmäßigerweise einen Akkumulator, ein Bedienelement zur Temperatureinstellung sowie einen Gleichrichter zur Gleichstromerzeugung. Sind mehrere Peltierelemente vorgesehen, ist es zweckmäßig, wenn diese einzeln bzw. separat ansteuerbar sind, um unterschiedliche Kühlkurven an unterschiedlichen Körperbereichen zu ermöglichen. Insbesondere wird das zumindest eine Peltierelement mit langsamen Pulsen von etwa 1 Hz, insbesondere etwa 0,7 Hz, besonders bevorzugt etwa 0,5 Hz oder weniger, angesteuert. Das Peltierelement kann aber auch mit einer schnelleren Pulsfrequenz angesteuert werden. Ferner kann es vorgesehen sein, dass die Vorrichtung bzw. die Steuereinheit mit einer App über ein Mobiltelefon, einen Tablet-PC oder dergleichen steuerbar ist. Eine solche App bietet verschiedene Kurven bzw. Daten für unterschiedliche Beschwerden an, aus welchen ein Patient bzw. Anwender wählen kann.

Wenn eine Temperatur genau geregelt werden soll, ist es weiter günstig, wenn zumindest ein Temperatursensor vorgesehen ist. Insbesondere ist es günstig, wenn mehrere, beispielsweise sechs, Temperatursensoren an unterschiedlichen Orten der Vorrichtung angeordnet sind. Die Temperatursensoren sind dabei bevorzugt nahe der Haut sowie an den Kühlmaterialien angeordnet. Insbesondere umfasst die zumindest teilweise flexible Leiterplatte einen oder mehrere Temperatursensoren. Ferner kann zumindest ein Temperatursensor zur Messung einer Umgebungstemperatur ausgebildet sein. Die Leiterplatte kann darüber hinaus weitere elektronische Bauteile umfassen, beispielsweise Sensoren zur Bestimmung einer Lichtintensität oder von Körperfunktionen. Des Weiteren kann es günstig sein, wenn Aktorikeinrichtungen vorgesehen sind, beispielsweise eine Vibrationseinrichtung zur Schläfenmassage.

Für Behandlungen, welche auch eine Zufuhr von Arzneimitteln und/oder Desinfektionsmittel erfordern, ist es zweckmäßig, wenn für eine Zuführung von Arzneimitteln eine Leitung vorgesehen ist. Diese ist bevorzugt an einer äußeren Oberfläche des Hautkontaktmaterials angeordnet. Dadurch sind bei einer Behandlung einer Körperregion neben einer Kühlung derselben gleichzeitig auch heilungsfördernde Substanzen zuführbar. Solche Substanzen bzw. Arzneimittel befinden sich bevorzugt in einem dafür vorgesehenen Tank und sind mit einer gezielten Steuerung an die Hautoberfläche abgebbar.

Es ist von Vorteil, wenn eine akustische Ausgabeeinrichtung und/oder eine Vibrationsausgabeeinrichtung vorgesehen sind. Diese können zur Ausgabe diverser Parameter wie Warnsignale und/oder einen Kühlungsfortschritt für einen Patienten oder einen behandelnden Arzt ausgebildet sein. Die Vibrationsausgabeeinrichtung umfasst vorzugsweise einen oder mehrere Vibrationsmotoren zur Erzeugung eines Vibrationssignals oder einer Vibrationssignalabfolge. Eine bestimmte Vibrationssignalabfolge bzw. Vibrationsmuster kann beispielsweise für einen bestimmten Parameter, Funktionen oder Warnsignale codieren. Somit dient die Vibrationsausgabeeinrichtung als Feedbacksystem für beispielsweise eine Überhitzung oder einen schwachen Akkumulator. Ferner kann vorgesehen sein, dass der zumindest eine Vibrationsmotor extern ansteuerbar ist, beispielsweise über eine Smartphoneapplikation. Dadurch kann beispielsweise während einer Kühlphase eine Vibration gezielt eingestellt werden, sodass diese anregend oder entspannend wirkt. Alternativ dazu können das Peltierelement und/oder der zumindest eine Vibrationsmotor durch ein vordefiniertes Programm angesteuert werden, welches einen vorbestimmten Temperaturverlauf und/oder eine vorbestimmte Vibrationsabfolge umfasst.

Es ist vorteilhaft, wenn eine Halterung zur Befestigung der Vorrichtung an einer Körperregion vorgesehen ist. Grundsätzlich ist es zwar zweckmäßig, eine Körperregion wie eine Nase oder eine Stirn in einer waagrechten Körperposition zu kühlen. Es kann jedoch auch vorgesehen sein, eine Körperregion in aufrechter Position zu kühlen. Hierfür ist die an die Vorrichtung anschließende Halterung zur Befestigung der Vorrichtung z. B. am Kopf ausgebildet. Dadurch ist ein zusätzlicher Halt bzw. eine besondere feste Fixierung gegeben, um auch eine Anwendung in sitzender oder stehender Körperposition bzw. eine portable Anwendung zu ermöglichen. Die Halterung ist insbesondere so ausgebildet, dass ein ungewolltes Verrutschen oder Wackeln der Vorrichtung vermieden ist. Die Vorrichtung kann grundsätzlich auch mit einer Form ausgebildet sein, welche ein selbsttätiges Halten derselben an einer Körperregion erlaubt, sodass die Vorrichtung selbst als Halterung dient.

Es ist von Vorteil, wenn die Vorrichtung zumindest teilweise schichtförmig aufgebaut ist. Insbesondere ist die Vorrichtung als thermisch konduktiver Schichtaufbau ausgebildet, welcher Wärme nach außen abgibt und zugleich flexibel ist. Dabei ist das Hautkontaktmaterial an einer Innenseite des Peltierelementes mit diesem verbunden, wobei das Hautkontaktmaterial in direktem bzw. unmittelbarem Kontakt mit der zu kühlenden Körperregion steht. Das zumindest eine Peltierelement ist in einem Isoliermaterial eingebettet, an welches das wärmeleitfähige Verbindungselement oder die Leiterplatte anschließt. Außenseitig am Verbindungselement ist die Leiterplatte angeordnet oder umgekehrt. Eine Außenseite abschließend ist zumindest ein Kühlkörper vorgesehen, welcher oberhalb des Peltierelementes angeordnet ist und als Aluminiumblech ausgebildet sein kann. Es kann auch ein zusätzliches starres Kühlelement vorgesehen sein, welches konduktiv mit dem Kühlkörper verbunden ist. Zusätzlich kann in derselben Ebene ein Drahtgeflecht und/oder Streckmetall vorgesehen sein. Außenseitig an der Vorrichtung ist erfindungsgemäß das Wärmeabführelement angeordnet, welches insbesondere als Platte aus einer Aluminiumlegierung ausgebildet ist, bevorzugt aus der Legierung AlMgSi1. Diese Legierung weist ähnliche mechanische Eigenschaften wie Federstahl auf und besitzt zusätzlich eine gute bzw. hohe Wärmeleitfähigkeit. Die Platte aus einer Aluminiumlegierung kann weiter außenseitig mit einem Lack dünnschichtig beschichtet sein. Hierbei ist es zweckmäßig, wenn die Lackschicht maximal 60 µm, insbesondere maximal 50 µm, bevorzugt 40 µm, dick ist. Die Lackschicht kann eine Grundierung und einem Lack umfassen. Alternativ oder zusätzlich zur Lackschicht kann die Platte eine Eloxalschicht aufweisen. Des Weiteren kann es vorgesehen sein, dass das Wärmeabführelement insbesondere etwa mittig Ausnehmungen wie Schlitze, Kreise oder dergleichen umfasst, sodass das Wärmabführelement in diesem Bereich besonders biegsam ausgebildet ist. Alternativ kann das Wärmabführelement auch bereichsweise dünner ausgebildet sein, um dieses über Sollbugstellen gezielt an eine beliebige Körperform anzupassen. Eine derartige Materialschwächung für die Sollbugstellen kann auch beispielsweise durch gefräste Taschen erfolgen. Günstig ist ferner, wenn die Vorrichtung zu einem großen Teil biegsam ausgebildet ist, sodass diese an die zu kühlende Körperregion anpassbar ist. Die einzelnen Schichten der Vorrichtung sind durch einen Wärmeleitkleber, einer Wärmeleitfolie und/oder einer Wärmeleitpaste miteinander verbunden, insbesondere verklebt, verschweißt, verlötet oder anderwärtig gefügt. Beispielsweise kann das Verbindungselement als Wärmeleitpaste ausgebildet sein. Alternativ dazu können die einzelnen Schichten aus einem vollen Stück gefräst sein.

Grundsätzlich ist die erfindungsgemäße Vorrichtung biegsam ausgebildet. Hierfür können die Sollbugstellen bzw. Sollbiegestellen im Allgemeinen beliebig ausgebildet sein. Auch eine gelenkige Verbindung einzelner Abschnitte der Vorrichtung ist dabei möglich, wenngleich dies zu einem komplexeren Aufbau führt.

Weitere Merkmale, Vorteile und Wirkungen ergeben sich aus den nachfolgend dargestellten Ausführungsbeispielen. In den Zeichnungen, auf welche dabei Bezug genommen wird, zeigen:
- Fig. 1: einen Schnitt durch eine erfindungsgemäße Vorrichtung;
- Fig. 2: eine erfindungsgemäße Vorrichtung;
- Fig. 3: eine weitere erfindungsgemäße Vorrichtung;
- Fig. 4: eine weitere erfindungsgemäße Vorrichtung;
- Fig. 5: eine schematische Darstellung einer erfindungsgemäßen Vorrichtung;
- Fig. 6: eine weitere schematische Darstellung einer erfindungsgemäßen Vorrichtung;
- Fig. 7: eine schematische Darstellung einer weiteren erfindungsgemäßen Vorrichtung;
- Fig. 8: eine schematische Darstellung eines Teils einer weiteren erfindungsgemäßen Vorrichtung.

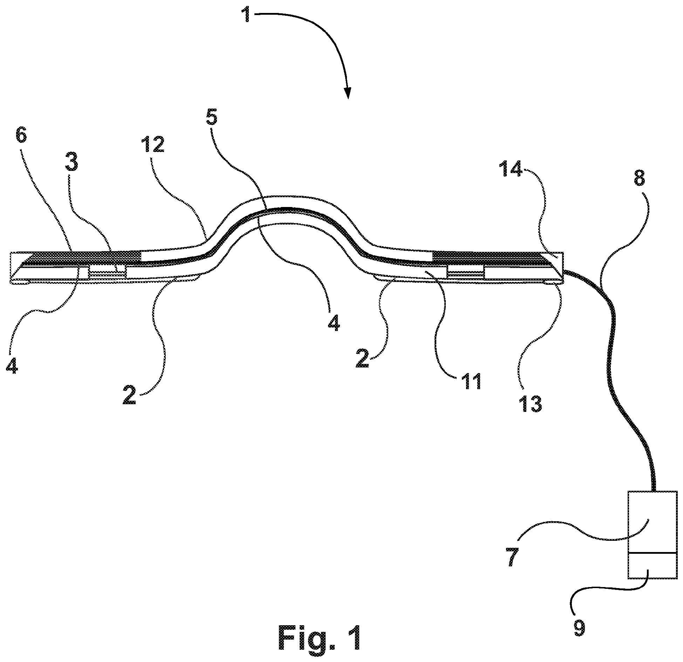

Fig. 1 zeigt einen Schnitt durch eine erfindungsgemäße Vorrichtung 1 zur Kühlung einer Körperregion, welche zur Kühlung eines Nasenbereiches ausgebildet ist. Die Vorrichtung 1 ist schichtförmig ausgebildet. An einer Unterseite der Vorrichtung 1 sind zwei Hautkontaktmaterialien 2 vorgesehen, welche an zu kühlenden Körperregionen anordenbar sind. Oberhalb derselben ist jeweils ein Peltierelement 3 vorgesehen, welches unmittelbar an das jeweilige Hautkontaktmaterial 2 anschließt. Die Peltierelemente 3 sind in einem flexiblen Isoliermaterial 11 angeordnet, welches aus Latex, Silicon oder einem anderen weichen Kunststoff, insbesondere aus einem thermoplastischen Elastomer wie einem thermoplastischen Polyurethan gebildet sein kann und eine kalte sowie warme Seite aufweist. An das Isoliermaterial 11 schließt ein als Wärmeleitfolie 5 ausgebildetes Verbindungselement an, wobei dieses als Grafitfolie oder grundsätzlich als sogenanntes Heatspreadermaterial ausgebildet ist. Grundsätzlich kann das Verbindungselement jedoch auch aus Wärmeleitpaste gebildet sein, welche das Peltierelement 3 mit einem Wärmeabführelement 12 verbindet. Außenseitig an der Wärmeleitfolie 5 ist eine zumindest teilweise flexible Leiterplatte 4 vorgesehen. Die Leiterplatte 4 umfasst mit Vorteil einen oder mehrere in Fig. 1 nicht gezeigte Temperatursensoren 18 und/oder andere elektronische Bauteile. Sowohl das Isoliermaterial 11, als auch die Wärmeleitfolie 5 und die Leiterplatte 4 sind zumindest teilweise flexibel bzw. biegsam ausgebildet, sodass sich diese an eine Form des Nasenbereiches anpassen. Weiter erstrecken sich diese Elemente im Wesentlichen über einen gesamten Bereich der Vorrichtung 1. Des Weiteren sind zwei starre Kühlkörper 6 vorgesehen, wobei zwischen diesen ein Wärmeabführelement 12 wie ein Drahtgeflecht angeordnet ist, welches die Vorrichtung 1 außenseitig abschließt. Anstatt des Drahtgeflechtes kann auch ein Streckmetall oder vollflächiges Aluminiumband als Wärmeabführelement 12 vorgesehen sein. Das Wärmeabführelement 12 ist unterschiedlich dick ausgebildet, sodass dieses individuell an eine Körperregion anpassbar ist. Ein innerer Bereich einer solchen Vorrichtung 1 kann auch starr ausgebildet sein. Um ein Verrutschen der Vorrichtung 1 zu vermeiden, sind zwei Antirutschelemente 13 vorgesehen. Diese liegen auf einer Hautoberfläche auf und können aus Latex gebildet sein. Prinzipiell kann die Vorrichtung 1 beliebig viele Antirutschelemente 13 umfassen. Die einzelnen Schichten der Vorrichtung 1 sind bevorzugt zumindest teilweise stoffschlüssig miteinander verbunden, beispielsweise mit Wärmeleitkleber. An zwei seitlichen Enden kann darüber hinaus ein Verbindungselement 14 angeordnet sein, welches aus Textil, Silicon oder einem weichen Kunststoff gebildet ist.

Um das Peltierelement 3 mit Energie bzw. Strom zu versorgen, ist eine bevorzugt als Akkumulator ausgebildete Energiequelle 7 vorgesehen, welche über ein Kabel 8 mittelbar mit der Vorrichtung 1 verbunden ist. Ferner ist eine Steuereinheit 9 vorgesehen, welche bevorzugt örtlich bei der Energiequelle 7 angeordnet sowie mit dieser verbunden ist und ebenfalls über das Kabel 8 mit der Vorrichtung in Verbindung steht. Die Energiequelle 7 kann jedoch auch direkt in der Vorrichtung 1 integriert sein.

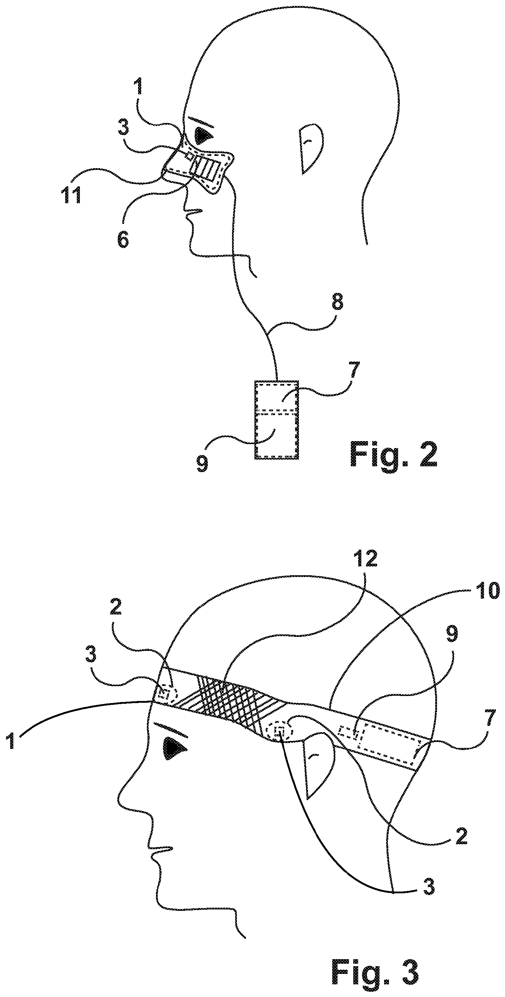

In Fig. 2 ist die erfindungsgemäße Vorrichtung 1 zur Kühlung einer Körperregion gemäß Fig. 1 gezeigt, welche am Nasenbereich eines Patienten angeordnet ist. Diese umfasst zwei Hautkontaktmaterialien 2 und zwei Peltierelemente 3. Die Hautkontaktmaterialien 2 sind in Fig. 2 nicht ersichtlich, da diese jeweils zwischen einem Peltierelement 3 und einer Haut des Nasenbereiches angeordnet sind. Die dargestellte Vorrichtung 1 umfasst weiter zwei starr ausgebildete Kühlkörper 6, welche beidseitig der Nase angeordnet sind. Allerdings kann auch nur ein einziger Kühlkörper 6 vorgesehen sein. In Fig. 2 ist darüber hinaus das Isoliermaterial 11 ersichtlich. Ferner sind die externe Energiequelle 7 und die externe Steuereinheit 9 gezeigt, welche über das Kabel 8 mit der Vorrichtung 1 verbunden sind. Die Energiequelle 7 kann beispielsweise im Nacken eines Patienten anordenbar sein, wobei diese fix mit der Vorrichtung 1 verbunden oder mit dieser verbindbar ist. Des Weiteren können Kordeln oder dergleichen für eine Größenverstellbarkeit der Vorrichtung 1 vorgesehen sein, welche hinter den Ohren des Patienten angeordnet werden können und somit die Vorrichtung 1 am Kopf des Patienten fixieren. Es kann auch eine nicht gezeigte Halterung 10 vorgesehen sein, um die Vorrichtung 1 am Kopf des Patienten zu befestigen. Eine Vorrichtung 1 zur Kühlung eines Nasenbereiches kann beispielsweise nach einer Nasenoperation, einer Tränensackentfernung, einer Lidoperation oder bei Schwellungen im Gesicht verwendet werden. Weiter ist die Vorrichtung 1 zur Förderung einer Durchblutung und zur Minderung von Fältchen oder Augenringen geeignet.

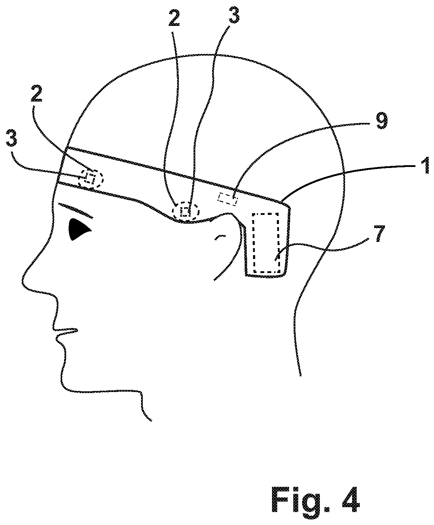

Fig. 3 und 4 zeigen jeweils eine weitere erfindungsgemäße Vorrichtung 1 zur Kühlung einer Körperregion. Diese sind jeweils zur Kühlung einer Stirn- und Schläfenregion ausgebildet und umfassen jeweils mehrere Hautkontaktmaterialien 2 und Peltierelemente 3. Eine Kühlung erfolgt mit Vorteil exakt am Stirnast und an den Wurzeln des Trigeminusnerves, wobei vier Kühlpunkte vorgesehen sind. Diese Stellen werden hierfür mit entsprechend an der Vorrichtung angeordneten Peltierelementen 3 gekühlt. Eine Energieversorgung der Peltierelemente 3 erfolgt über eine Energiequelle 7. Neben der Energiequelle 7 ist eine Steuereinheit 9 vorgesehen, über welche die Vorrichtung 1 steuerbar und regelbar ist. Die Energiequelle 7 und die Steuereinheit 9 sind über Kabel 8 mit den Peltierelementen 3 verbunden, wobei Kabel 8 innerhalb der Vorrichtung 1 geführt und deshalb in Fig. 3 nicht gezeigt sind. Die Vorrichtung 1 umfasst weiter ein Wärmeabführelement 12, welches biegbar ausgebildet sein kann. Besonders bevorzugt ist das Wärmeabführelement 12 als ein Aluminiumblech aus der Legierung AlMgSi1 gebildet, welche ähnliche Eigenschaften wie Federstahl aufweist und zudem Wärme gut leitet. Dadurch ist die Vorrichtung 1 an verschiedene Kopfformen und Kopfgrößen anpassbar. Ferner kann die Vorrichtung 1 beispielsweise zwischen Stirn und Schläfe eine Sollbugstelle 21 aufweisen. Darüber hinaus ist die in Fig. 3 gezeigte Vorrichtung 1 über eine Halterung 10 am Kopf eines Patienten befestigbar. Die Halterung 10 kann aus einem Textil, Silicon oder einem weichen Kunststoff gebildet sein und fixiert die Vorrichtung so am Kopf, dass diese nicht verrutscht. Die in Fig. 4 gezeigte Vorrichtung 1 ist im Gegensatz dazu so ausgebildet, dass diese durch eine Spangenwirkung des als Aluminiumblech ausgebildeten Wärmeabführelementes 12 selbststätig ohne Halterung 10 am Kopf eines Patienten oder Anwenders fixierbar ist. Diese Vorrichtung 1 umfasst einen Bügel mit einer Energiequelle 7, wobei der Bügel so hinter einem Ohr des Patienten anordenbar ist, dass die Vorrichtung 1 am Kopf des Patienten fixiert ist. Die Vorrichtung 1 weist also eine ähnliche Form wie eine Brille auf und ist flexibel ausgebildet, sodass die Vorrichtung 1 aufsetzbar ist. Weiter kann die Vorrichtung 1 neben einer Sollbugstelle 21 zwischen Stirn und Schläfe auch im Bereich des Ohres eine Sollbugstelle 21 aufweisen. Es kann auch vorgesehen sein, eine Vorrichtung 1 in eine optische Brille, Sonnenbrille oder Virtual-Reality-Brille bzw. in einen Helm oder andere Wearables zu integrieren.

Eine in Fig. 3 und 4 gezeigte Vorrichtung 1 zur Kühlung einer Stirn- und Schläfenregion kann unterschiedliche Beschwerden positiv beeinflussen: Migräne, Kopfschmerzen, Stress, Fieber, Konzentrationsschwierigkeiten, Hitzewallungen, Kreislaufproblemen, Müdigkeit, Energieverlust, Sinusitis und weitere Beschwerden. Darüber hinaus kann eine solche Vorrichtung 1 von Astronauten im Weltraum verwendet werden, da diese öfters an Schmerzen im Kopfbereich sowie an Brechreiz und Orientierungsschwäche leiden. Eine weitere Verwendung ergibt sich für Kampfpiloten, Scharfschützen, Fluglotsen und ähnliche Berufsgruppen. Es kann auch vorgesehen sein, dass die einzelnen Elemente der Vorrichtung 1 in einer Hülle aus z. B. Kunststoff eingefasst sind, um das Peltierelement 3 und dessen thermische Übergänge vor mechanischer Beanspruchung zu schützen. Eine Hülle aus Kunststoff weist mit Vorteil eine entsprechende Flexibilität auf, sodass die Vorrichtung 1 flexibel ausgebildet ist. Insbesondere eignet sich hierzu ein Kunststoff mit einer Shore-Härte im Bereich von etwa 50 Shore bis 70 Shore, sodass ein Optimum aus Anpassbarkeit und Gebrauchseigenschaften gegeben ist.

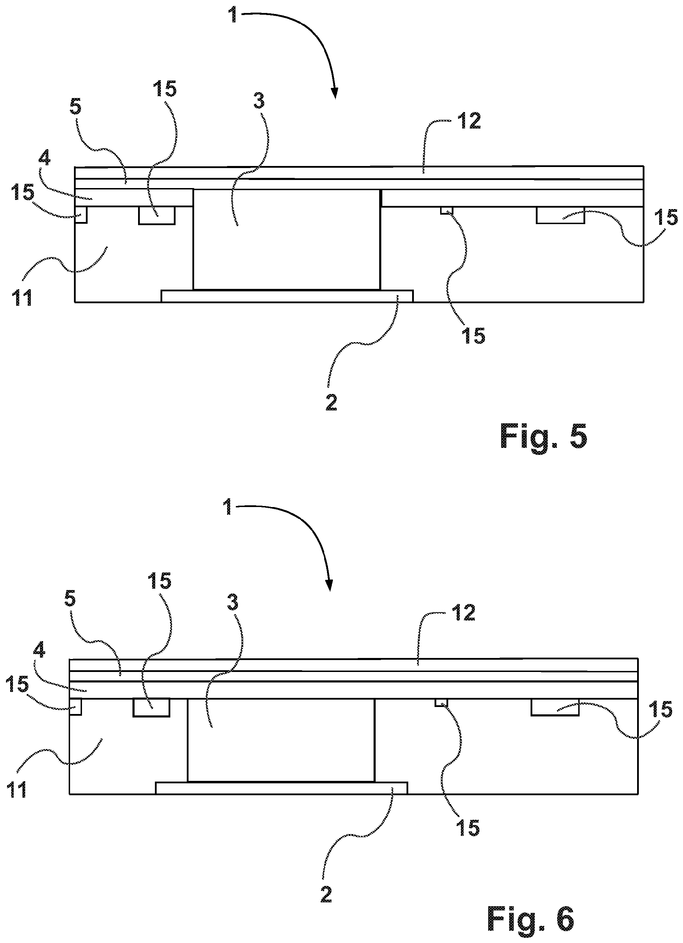

In Fig. 5 ist eine schematische Darstellung einer erfindungsgemäßen Vorrichtung 1 gezeigt, in welcher ein schichtweiser Aufbau der Vorrichtung 1 ersichtlich ist. Um effiziente Wärmeübergänge sicherzustellen, sind einzelne Schichten der Vorrichtung 1 mit einem Wärmeleitkleber, einer selbstklebenden Wärmeleitfolie, einem Wärmeleitpad 16 und/oder einer Wärmeleitpaste miteinander verbunden, insbesondere miteinander verklebt. Die schichtförmig aufgebaute Vorrichtung 1 umfasst ausgehend von einer auf einer der zu kühlenden Körperregion aufliegenden Seite: ein als eloxiertes oder versilbertes Aluminiumplättchen, zuerst versilbertes und anschließend rhodiniertes Kupferplättchen oder vernickelt und vergoldetes Kupferplättchen ausgebildetes Hautkontaktmaterial 2, ein in ein Isoliermaterial 11 eingebettetes Peltierelement 3, eine Leiterplatte 4 mit mehreren elektronischen Bauteilen 15, ein als Wärmeleitfolie 5 ausgebildetes Verbindungselement und ein Wärmeabführelement 12, vorzugsweise aus Aluminium oder einer Aluminiumlegierung. Das Kupferplättchen kann beispielsweise als Tiefziehstanzteil gefertigt sein.

Auch Fig. 6 zeigt eine schematische Darstellung einer erfindungsgemäßen Vorrichtung 1. Diese entspricht im Wesentlichen der Darstellung in Fig. 5. Im Unterschied zu einer in Fig. 5 gezeigten Vorrichtung 1 verläuft bei dieser Vorrichtung 1 die Leiterplatte 4 zwischen einer warmen Seite des Peltierelementes 3 und der Wärmeleitfolie 5 bzw. dem Wärmeabführelement 12 durch. Somit erfolgt hier eine Wärmeabführung über Leiterplatte 4 an die Wärmeleitfolie 5 und das Wärmeabführelement 12.

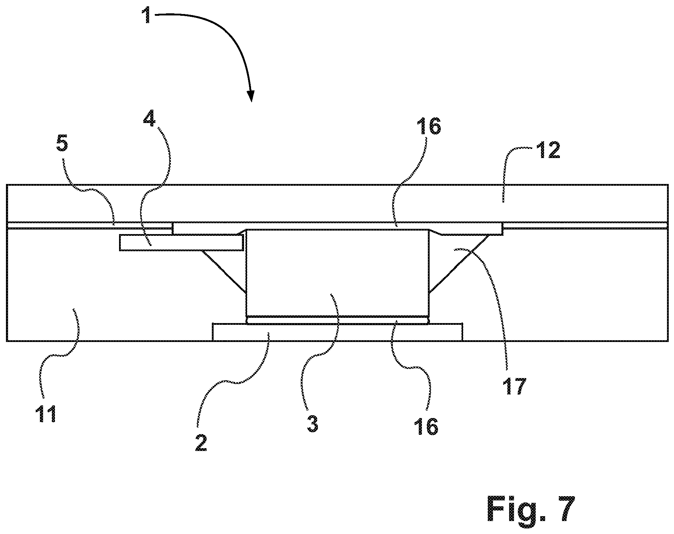

Eine schematische Darstellung einer weiteren erfindungsgemäßen Vorrichtung 1 ist in Fig. 7 gezeigt. Diese ist wiederum schichtweise aufgebaut und umfasst ein Hautkontaktmaterial 2, ein Peltierelement 3, eine insbesondere vorwiegend aus Kupfer gebildete Leiterplatte 4, eine optionale Wärmeleitfolie 5 und ein Wärmeabführelement 12. Das Hautkontaktmaterial 2 ist über ein Wärmeleitpad 16 mit dem Peltierelement 3 verbunden, welches auch über ein Wärmeleitpad 16 mit dem Wärmeabführelement 12 verbunden ist. Des Weiteren ist ein Isoliermaterial 11 vorgesehen. Die Vorrichtung 1 umfasst weiter einen Hohlraum 17 bzw. mit Luft gefüllten Raum, um eine kalte und warme Seite des Peltierelementes 3 thermisch voneinander zu entkoppeln.

Es kann weiter vorgesehen sein, dass in der gesamten Vorrichtung 1 eine elektrische Leiterplatte 4 integriert ist, welche aus starren und flexiblen Teilen aufgebaut ist. Dadurch kann der elektronische Schaltkreis hochintegriert in die Vorrichtung 1 eingebaut werden. Eine solche Leiterplatte 4 weist eine Flexibilität an den gewünschten Stellen auf, wodurch starke Verbiegungen ohne Beschädigung möglich sind. An der Stirnseite der Vorrichtung 1 wird die Abwärme des Peltierelements 3 über einen flexiblen Teil der Leiterplatte 4 abgeführt, welcher zwischen zwei Wärmeleitpads angeordnet ist. Durch die sehr dünne Ausführung von etwa 130 µm des flexiblen Teiles der elektrischen Leiterplatte 2 wird die gewünschte Wärmeabführung über die Wärmeleitpads an die Oberfläche des Wärmeabführelementes 12 kaum beeinträchtigt. Die Leiterplatte 4 umfasst weiter Kupferbahnen, welche durch den flexiblen und starren Teil der Leiterplatte verlaufen und elektrisch miteinander verbunden sind. Dadurch sind weitere elektrische Bauteile wie Temperatursensoren 18 auf die Leiterplatte 4 auflötbar. Da sich die auf der Leiterplatte 4 vorhandenen Kupferbahnen ebenfalls erwärmen, wird auch ein darauf aufgelöteter Temperatursensor 18 diese Temperatur annehmen. Somit sind Temperaturen an den gewünschten Stellen einfach messbar, da die Sensoren auf dem flexiblen Teil der Leiterplatte 4 thermische und elektrische Verbindung umfassen. Aufgrund der Flexibilität können Teile der flexiblen Leiterplatte 4 so konstruiert sein, dass diese an gewünschten Messpunkten gebogen und teilweise in den thermischen Schichtaufbau eingeklemmt werden können. Außerdem ist eine gesamte Wärmeabführung verbessert, wenn der flexible Teil der elektrischen Leiterplatte 4 zu einem großen Teil aus Kupferbahnen besteht, da die Wärme auch entlang dieser Kupferbahnen bzw. Kupferleitungen der elektrischen Leiterplatte 4 abführbar ist. Der flexible Teil der Leiterplatte 4 ist folglich grundsätzlich für die Elektronik ausgebildet, wobei die Leiterplatte 4 aufgrund der Kupferbahnen auch gut thermisch leitfähig ist, weshalb sich diese auch hervorragend für Temperaturmessungen eignet.

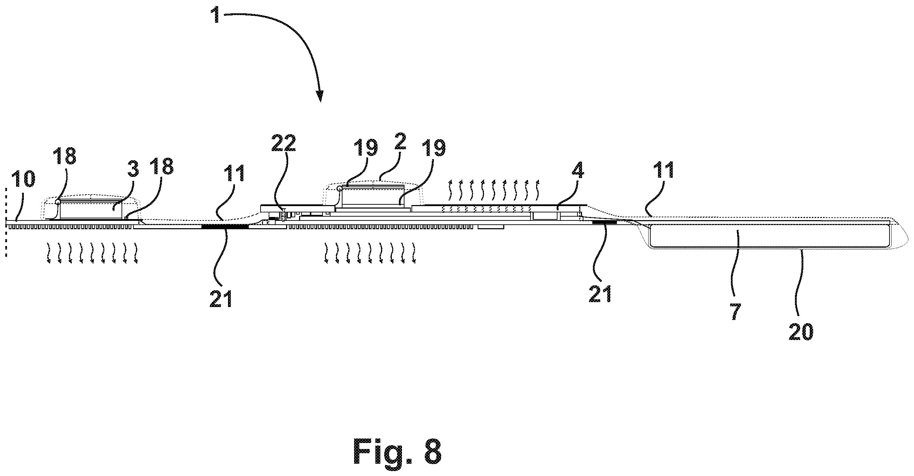

In Fig. 8 ist eine erste Hälfte einer weiteren Ausführungsform einer Vorrichtung 1 dargestellt. Die zweite Hälfte der Vorrichtung 1 bzw. die vollständige Vorrichtung 1 ergibt sich durch Spiegelung der ersten Hälfte an einer Achse, welche in Fig. 8 durch eine gestrichelte Linie dargestellt ist, bzw. durch Verlängerung der Vorrichtung 1 über diese Linie hinaus. Die zweite Hälfte der Vorrichtung 1 ist im Wesentlichen analog zur ersten Hälfte der Vorrichtung 1 ausgebildet, umfasst jedoch nicht notwendigerweise sämtliche dargestellten Komponenten. Hierbei umfasst die Halterung 10 eine Blechkonstruktion, insbesondere aus einem AlMgSi1-Blech. Dabei können Einzelteile der Halterung 10, beispielsweise Bleche, unterschiedliche Größen und/oder Aussparungen aufweisen, um eine Oberfläche zu maximieren, welche in direktem Kontakt mit einer Umgebungsluft steht. Die einzelnen Bleche der Halterung 10 können vorzugsweise verklebt, verschraubt, verlötet, verschweißt oder anderweitig verbunden sein. Alternativ dazu kann die Halterung 10 aus einem Stück hergestellt sein, beispielsweise durch Fräsen und/oder andere spanabhebende Verfahren. Aktive Bereiche einer solchen Vorrichtung 1 können jeweils ein Peltierelement 3 und zumindest einen Temperatursensor 18 umfassen. Darüber hinaus kann ein Hautkontaktmaterial 2 vorgesehen sein, welches insbesondere als bevorzugt vergoldetes Hautkontaktplättchen ausgebildet ist. Das Hautkontaktplättchen kann ein gut wärmeleitendes Material, insbesondere Kupfer, umfassen. Unterhalb des Hautkontaktmaterials 2 kann beispielsweise zumindest ein Thermal-Pad 19 angeordnet sein. An einer Oberseite der Vorrichtung 1 kann insbesondere in einem flexiblen Bereich, beispielsweise im Bereich von Sollbugstellen 21, ein Isoliermaterial 11 bzw. ein flexibles Kunststoffteil, insbesondere aus einem thermoplastischen Elastomer, angeordnet sein. Dieses flexible Kunststoffteil dient insbesondere zur weichen Auflage an einem Körperteil. Die elektrische Leiterplatte 4 ist bevorzugt mit thermischen Vias ausgebildet, da somit Wärme durch die Leiterplatte 4 transportiert und körperseitig abgegeben werden kann. Eine Abwärme ist hierbei durch geschwungene Pfeile angedeutet. Körperseitig ist hierbei ein und umgebungsseitig sind zwei hauptsächliche Abwärmebereiche angedeutet. Die Leiterplatte 4 selbst kann vorzugsweise aus einem eloxierten Blech ausgebildet sein. Vorzugsweise weist dieses Blech eine Dicke von etwa 0,2 mm auf. Zur Fixierung kann die Leiterplatte 4 beispielsweise mit zumindest einer Schraube 22 mit der Halterung 10 verbunden sein. Darüber hinaus ist eine Energiequelle 7, insbesondere ein Lithium-Polymer-Akkumulator, vorgesehen, welcher in ein Akkumulatorgehäuse 20 eingefasst ist. Das Akkumulatorgehäuse 20 kann vorzugsweise aus einem Kunststoff, beispielsweise aus einem Acrylnitril-Butadien-Styrol-Copolymer (ABS), thermoplastisches Polyurethan (TPU) oder dergleichen ausgebildet sein oder eines oder mehrere dieser Materialien umfassen. Bei der Vorrichtung 1 kann beispielsweise vorgesehen sein, dass nur eine Energiequelle 7 vorgesehen ist. Dementsprechend kann die zweite, nicht dargestellte Hälfte ohne Energiequelle ausgebildet sein. Um eine länger andauernde Energieversorgung bereitzustellen, kann vorgesehen sein, dass beide Hälften eine Energiequelle 7 aufweisen.

Eine erfindungsgemäße Vorrichtung 1 kann neben bereits genannten Anwendungen auch zur Kühlung, Erwärmung und/oder Behandlung für beispielsweise folgende weitere Körperregionen und/oder Beschwerden verwendet werden:
- Insektenstiche;
- am Haarbereich bei einer Chemotherapie zum Schutz vor Haarausfall;
- Wund- und Narbenkühlung;
- Spannungskopfschmerz im Nackenbereich;
- Kiefer- und Zahnschmerzen;
- Beschwerden im Bauchbereich;
- Schwellungen bei Knochenbruch;
- Im Hodenbereich zur Verbesserung einer Spermienqualität;
- Nebenhodenentzündung;
- Hodenprellung;
- Hodenruptur;
- an Gelenken;
- Sportverletzung;
- Muskelschmerzen;
- Thrombose;
- Multiple Sklerose;
- Müdigkeit.

## Patentansprüche

1. Vorrichtung (1) zur Kühlung und/oder Erwärmung einer Körperregion, bevorzugt eines Kopfbereiches, umfassend zumindest ein wärmeleitfähiges Hautkontaktmaterial (2) und zumindest ein Peltierelement (3), wobei das Peltierelement (3) mit dem Hautkontaktmaterial (2) verbunden ist, wobei das Hautkontaktmaterial (2) aus einer biegsamen metallischen Folie oder aus mehreren kleinen starren Blechelementen ausgebildet ist und ein wärmeleitfähiges Verbindungselement und ein Wärmeabführelement (12) vorgesehen sind, wobei über das Verbindungselement über eine Außenseite des Peltierelementes (3) abgegebene Wärme auf das Wärmeabführelement (12) verteilbar ist, wobei das Wärmeabführelement (12) außenseitig an der Vorrichtung (1) angeordnet und biegbar ausgebildet ist, und dass die Vorrichtung (1) frei von flüssigem Wärmetransportmittel ausgebildet ist.

2. Vorrichtung (1) nach Anspruch 1, **dadurch gekennzeichnet, dass** zumindest eine Leiterplatte (4) vorgesehen ist.

3. Vorrichtung (1) nach Anspruch 1 oder 2, **dadurch gekennzeichnet, dass** zumindest ein Kühlkörper (6) vorgesehen ist.

4. Vorrichtung (1) nach einem der Ansprüche 1 bis 3, **dadurch gekennzeichnet, dass** zum Betrieb des Peltierelementes (3) eine Energiequelle (7) vorgesehen ist, wobei die Energiequelle (7) insbesondere über Kabel (8) mit dem Peltierelement (3) verbunden ist.

5. Vorrichtung (1) nach einem der Ansprüche 1 bis 4, **dadurch gekennzeichnet, dass** eine Steuereinheit (9) vorgesehen ist.

6. Vorrichtung (1) nach einem der Ansprüche 1 bis 5, **dadurch gekennzeichnet, dass** zumindest ein Temperatursensor (18) vorgesehen ist.

7. Vorrichtung (1) nach einem der Ansprüche 1 bis 6, **dadurch gekennzeichnet, dass** für eine Zuführung von Arzneimittel eine Leitung vorgesehen ist.

8. Vorrichtung (1) nach einem der Ansprüche 1 bis 7, **dadurch gekennzeichnet, dass** eine akustische Ausgabeeinrichtung und/oder eine Vibrationsausgabeeinrichtung vorgesehen sind.

9. Vorrichtung (1) nach einem der Ansprüche 1 bis 8, **dadurch gekennzeichnet, dass** eine Halterung (10) zur Befestigung der Vorrichtung (1) an einer Körperregion vorgesehen ist.

10. Vorrichtung (1) einem der Ansprüche 1 bis 9, **dadurch gekennzeichnet, dass** die Vorrichtung (1) zumindest teilweise schichtförmig aufgebaut ist.

## Claims

1. Device (1) for cooling and/or heating a body region, preferably a head region, comprising at least one thermally conductive skin contact material (2) and at least one Peltier element (3), the Peltier element (3) being connected to the skin contact material (2), wherein the skin contact material (2) is made from a bendable metallic film or from multiple small rigid metal sheet elements and wherein a a thermally conductive connecting element and a heat dissipation element (12) are provided, wherein heat emitted via an outer side of the Peltier element (3) is configured to be distributed to the heat dissipation element (12) via the connecting element, wherein the heat dissipation element (12) is arranged on an outer side of the device (1) and embodied to be bendable, and wherein the device (1) is embodied to be free of liquid heat transfer media.

2. Device (1) of claim 1 **characterized in that** at least one circuit board is provided.

3. Device (1) of claim 1 or 2 **characterized in that** at least one cooling body (6) is provided.

4. Device (1) of one of the claims 1 to 3 **characterized in that** an energy source (7) is provided for operating the Peltier element (3), wherein the energy source (7) is connected to the Peltier element (3) in particular via cables.

5. Device (1) of one of the claims 1 to 4 **characterized in that** a control unit (9) is provided.

6. Device (1) of one of the claims 1 to 5 **characterized in that** at least one temperature sensor (18) is provided.

7. Device (1) of one of the claims 1 to 6 **characterized in that** a line is provided for a supply of pharmaceuticals.

8. Device (1) of one of the claims 1 to 7 **characterized in that** an acoustic output device and/or a vibration output device are provided.

9. Device (1) of one of the claims 1 to 8 **characterized in that** a mount (10) is provided for securing the device (1) to a body region.

10. Device (1) of one of the claims 1 to 9 **characterized in that** the device (1) is constructed in an at least partially layered manner.

## Revendications

1. Dispositif (1) pour refroidir et/ou chauffer une zone du corps, de préférence une zone de la tête, comprenant au moins un matériau de contact cutané thermoconducteur (2) et au moins un élément Peltier (3), l'élément Peltier (3) étant relié au matériau de contact cutané (2), dans lequel le matériau de contact cutané (2) est constitué d'un film métallique pliable ou de plusieurs petits éléments de feuilles métalliques rigides et dans lequel un élément de connexion thermoconducteur et un élément de dissipation de chaleur (12) sont prévus, dans lequel la chaleur émise par un côté extérieur de l'élément Peltier (3) est configurée pour être distribuée à l'élément de dissipation thermique (12) via l'élément de connexion, dans lequel l'élément de dissipation thermique (12) est disposé sur un côté extérieur du dispositif (1) et est conçu pour être pliable, et dans lequel le dispositif (1) est conçu pour être dépourvu de fluide de transfert de chaleur.

2. Dispositif (1) de la revendication 1 **caractérisé par** la présence d'au moins une carte de circuit imprimé.

3. Dispositif (1) de la revendication 1 ou 2 **caractérisé par** la présence d'au moins un corps de refroidissement (6).

4. Dispositif (1) de l'une des revendications 1 à 3 **caractérisé en ce qu'**une source d'énergie (7) est prévue pour faire fonctionner l'élément Peltier (3), la source d'énergie (7) étant reliée à l'élément Peltier (3) notamment par des câbles.

5. Dispositif (1) de l'une des revendications 1 à 4 **caractérisé par** la présence d'une unité de commande (9).

6. Dispositif (1) de l'une des revendications 1 à 5 **caractérisé par** la présence d'au moins un capteur de température (18).

7. Dispositif (1) de l'une des revendications 1 à 6 **caractérisé par le fait qu'**une ligne est prévue pour l'approvisionnement en produits pharmaceutiques.

8. Dispositif (1) de l'une des revendications 1 à 7 **caractérisé par** la présence d'un dispositif de sortie acoustique et/ou d'un dispositif de sortie vibratoire.

9. Dispositif (1) de l'une des revendications 1 à 8 **caractérisé en ce qu'**une monture (10) est prévue pour fixer le dispositif (1) à une zone du corps.

10. Dispositif (1) de l'une des revendications 1 à 9 **caractérisé par le fait que** le dispositif (1) est construit au moins partiellement en couches.
